Europäisches Patentamt

European Patent Office

Office européen des brevets

(11) Publication number: **0 244 825 B1**

# EUROPEAN PATENT SPECIFICATION

(45) Date of publication of patent specification: **07.10.92** (51) Int. Cl.⁵: **G01N 33/52**, C12Q 1/60

(21) Application number: **87106500.9**

(22) Date of filing: **05.05.87**

The file contains technical information submitted after the application was filed and not included in this specification

---

(54) **Dry-type analytical element for cholesterol.**

---

(30) Priority: **09.05.86 JP 105977/86**
**20.05.86 JP 115035/86**

(43) Date of publication of application:
**11.11.87 Bulletin 87/46**

(45) Publication of the grant of the patent:
**07.10.92 Bulletin 92/41**

(84) Designated Contracting States:
**DE GB**

(56) References cited:
EP-A- 0 019 253       EP-A- 0 091 026
EP-A- 0 176 357       FR-A- 2 266 170
GB-A- 1 318 568       US-A- 3 983 005
US-A- 3 992 158       US-A- 4 042 329

**PATENT ABSTRACTS OF JAPAN, vol. 10, no. 198 (P-476)[2254], 11 June 1986**

**ANALYTICAL CHEMISTRY, vol. 55, no. 4, April 1983, American Chemical Society, US; B.WALTER, pp. 498a-514a**

(73) Proprietor: **FUJI PHOTO FILM CO., LTD.**
**210 Nakanuma Minami Ashigara-shi Kanagawa 250-01(JP)**

(72) Inventor: **Arai, Fuminori, c/o Fuji Photo Film Co.Ltd.**
**11-46, Senzui 3-chome Akasa-shi, Saitama(JP)**

(74) Representative: **Kraus, Walter, Dr. et al Patentanwälte Kraus, Weisert & Partner Thomas-Wimmer-Ring 15 W-8000 München 22(DE)**

---

**Description**

This invention relates to a dry-type analytical element for the determination of cholesterol particularly total cholesterol including the cholesterol in a bound state in a biological body fluid such as blood or other liquid samples.

The quantitative analysis of cholesterol using cholesterol oxidase is known (Japanese Patent KOKOKU No. 54-8318). In this method, cholesterol is oxidized in the presence of cholesterol oxidase to produce hydrogen peroxide and cholestenone, and one of the products is determined by a known method.

On the other hand, it is said that cholesterol exists in blood as protein-bound cholesterol ester and free cholesterol ester in addition to free cholesterol. Thus, in order to determine total cholesterol concentration, the cholesterol in the above bound states should be converted to free cholesterol.

The combination of cholesterol esterase with cholesterol oxidase for the purpose of total cholesterol determination is disclosed in USP 3,925,164. In this method, the cholesterol in a bound state is liberated in the presence of cholesterol esterase, and oxidized in the presence of cholesterol oxidase.

In order to determine $H_2O_2$, Trinder reagent is useful, but the presense of bilirubin gives an error to the analytical result. As the means to remove the interference of bilirubin, the addition of a ferrocyanate is disclosed in USP 4,291,121. However, when a ferrocyanate is added to a dry-type analytical element containing cholesterol esterase, cholesterol oxidase, peroxidase and Trinder reagent (or a similar reagent system to detect hydrogen peroxide), the stability of the analytical element during storage is remarkably lowered.

It is also known that, in the case of using cholesterol esterase, another means to decompose the bond between protein and cholesterol ester is necessary. As this means, protease is employed together with lipase in USP 3,869,349, and a surfactant such as polyethylene glycol alkyl ether is employed together with cholesterol esterase in USP 3,925,164. Furthermore, it is disclosed in USP 4,275,151 and USP 4,274,152 that polyethylene glycol alkylphenyl ethers (alkylphenoxypolyethoxyethanol) having less than 20 ethylene glycol units are effective.

However, when the polyethylene glycol alkyl phenyl ether having less than 20 ethylene glycol units was incorporated into a porous spreading layer of a dry-type analytical element, spreading area of a liquid sample in the spreading layer became too broad. As a result, some problems such as lowering of sensitivity in colorimetry, lowering of quantitative spreading (proportionality between supplied amount of liquid and spreading area) and insufficient wettability of coating solution to the spreading layer happened. In addition, when this surfactant was incorporated in the layer existing between the spreading layer and a support and being composed of a hydrophilic polymer binder, such as a water absorption layer, a reagent layer, a light-blocking layer or an adhesive layer, wetting between this layer and another layer was liable to be insufficient. This brought unevenness in coating.

US-A-3 983 005 describes an integral analytical element for analysis of liquids for their cholesterol content.

The element is of the type which comprises at least two superposed layers including a spreading layer and a reagent layer in fluid contact and, optionally, a support. Cholesterol oxidase and a composition for the hydrolyses of cholesterol esters comprising lipase having cholesterol esterase activity and protease and included in the element such that cholesterol esters contained in a sample to the spreading leayer are saponified to free cholesterol and free cholesterol is decomposed in the presence of cholesterol oxidase to produce a detectable change related to the total chlesterol content of the sample.

EP-A-0 176 357 describes an analytical element for the colorimetric determination of total cholesterol in aqueous liquids, such as biological fluids. This element comprises an absorbent material and critical amounts of cholesterol ester hydrolase (from 1500 to 12,000 I.U./m$^2$) and a nonionic surfactant (from 5 and upt to, but less than 11 g/m$^2$).

An object of the invention is to provide a dry-type analytical element for the determination of cholesterol, having a good sensitivity comparable to the conventional dry-type analytical element containing cholesterol esterase, cholesterol oxidase, peroxidase and Trinder reagent, and having a long shelf life.

Another object of the invention is to provide a dry-type analytical element for the determination of cholesterol having a suitable spreading area in a definite time and having a high sensitivity to total cholesterol in colorimetry.

Still another object of the invention is to provide a dry-type analytical element for the determination of cholesterol having a good quantitative spreading and capable of uniform coating of a coating solution in its preparation.

As the dry-type analytical element for the determination of cholesterol achieving such objects, the present invention provides a dry-type analytical element for the determination of cholesterol comprising at

2

least one liquid-permeable layer and having a porous spreading layer, which is a liquid permeable layer, and containing an enzyme having the hydrolytic activity against cholesterol ester, a bile acid compound and cholesterol dehydrogenase in the same or different liquid-permeable layers.

The enzyme having the hydrolytic activity against cholesterol ester (cholesterol ester hydrolase) includes cholesterol esterase and lipase. Cholesterol esterase may be obtained from an animal organ or from a microorganism such as Candida rugosa, Actinomycetes, Streptomyces, Penicillium, etc. These microorganisms are disclosed in USP 3,925,164. Lipase may be of plant origin such as wheat germ, animal origin such as pancreas or microbial origin such as Candida rugosa. However, the lipase obtained from Chromobacterium viscosum is preferable. Lipase may be combined with cholesterol esterase to use.

In order to raise the reaction rate described dehydrogenase, an oxidized form coenzyme, a dye precursor and an electron transporting carrier substance are used. In this coloring utilizing this reaction, a dye is produced from a dye precursor. The bile acid includes deoxycholic acid, taurocholic acid, glycocholic acid, taurodeoxycholic acid and glycodeoxycholic acid. The bile acid and its derivative may be a salt such as sodium salt, potassium salt or lithium salt. Among the above compounds, deoxycholic acid and its salt is the most preferable. Hereafter, the bile acid, its derivative and these salts are referred to as bile acid compounds. Effect of the bile acid compound remarkably appears in the case of the coloring system of cholesterol dehydrogenase described below.

Cholesterol is detected by the following coloring system.

In this coloring system, cholesterol dehydrogenase is employed. The reagent composition for this coloring system is composed of cholesterol dehydrogenase, an oxidized form coenzyme, a color precursor and an electron transporting material. In this coloring system, cholesterol is dehydrogenated in the presence of cholesterol dehydrogenase and an oxidized form coenzyme to produce cholestenone, and at that time, the oxidized form coenzyme is converted to its reduced form. By utilizing this reaction, a color is produced from a color precursor.

Preferable cholesterol dehydrogenases are NAD- or NADP-dependent type disclosed in Japanese Patent KOKAI Nos. 53-56090, 58-89200 and 61-108400.

As the oxidized form coenzyme, oxidized form nicotinamide adenine dinucleotide (NAD) and oxidized form nicotinamide adenine dinucleotide phosphate (NADP) are usable.

The color precursor includes various tetrazolium salts such as 3,3'-(3,3'-dimethoxy-4,4'-biphenylene)bis-[2-(p-nitrophenyl)-5-phenyltetrazolium chloride] (NBT), 3,3'-(3,3'-dimethoxy-4,4'-biphenylene)bis[2,5-diphenyltetrazolium chloride] (BT), 3-(4',5'-dimethyl-2-triazolyl)-2,4-diphenyltetrazolium bromide (MTT), 2-(p-iodophenyl)-3-(p-nitrophenyl)-5-phenyltetrazoliumchloride (INT), 2,2',5,5'-tetra(p-nitrophenyl)-3,3'-(3,3'-dimethoxy-4,4'-biphenylene)ditetrazolium chloride (TNBT), 2,3,5-triphenyltetrazolium chloride (TT) and 3,3'-(4,4'-biphenylene)bis[2,5-diphenyltetrazolium chloride] (NT). Among these, NBT (common name; Nitrotetrazolium Blue) is preferable.

The electron carrier is diaphorase (E.C. 1, 6, 4, 3), N-methylphenazine methosulfate compound such as N-methylphenazine methoxysulfate or 1-methoxy-N-methylphenazine methosulfate, Meldra Blue, Methylene Blue or the like. Among them, diaphorase and N-methylphenazine methosulfate are preferable.

In this coloring system, it is preferable that the dye precursor is separated from the carrier , and they are incorporated in different layers from each other. Other components may be incorporated in either of the above layers or other suitable layers. By the above separation, gradual degradation of the reagent composition and fogging during storage decrease. As a result, sensitivity and accuracy are raised.

Other reagents may be added according to kind of the sample etc. Such reagents include a nonionic surfactant, a sensitivity moderator and a buffer.

The nonionic surfactant includes a polyhydric alcohol ester ethylene oxide adduct (condensate), a polyethylene glycol monoester, a polyethylene glycol diester, a higher alcohol ethylene oxide adduct (condensate) and an alkylphenol ethylene oxide adduct (condensate). Examples of the nonionic surfactant are disclosed in USP 3,925,164, USP 3,983,005, USP 4,275,152 and USP 4,275,151, and include,

POE (10) sorbitan monooleate
PEG (400) monostearate
Lauryl alcohol EO 10 moles condensate
POE (10) octylphenyl ether
POE (15) octylphenyl ether
POE (12) nonylphenyl ether
Hydroxypolyethoxydodecane
(Note)
    POE:     Polyethylene oxide
    PEG:     Polyethylene glycol

3

EO : Ethylene oxide

The number in parentheses represents condensation number of ethylene oxide units

As the nonionic surfactant, an alkylphenoxypolyglycidol is preferable. Preferable carbon number of the alkyl group is 4 to 20. The above benzene ring may be substituted by two or more alkyl groups, that is, a dialkylphenoxypolyglycidol is also included. The benzene ring may contain other groups such as an alkoxyl group or a halogen atom. The number of glycidol units is usually 7 to 20.

The content per square meters of each component in the analytical element of the invention is as follows:

The content of cholesterol esterase is 500 to 50,000 IU preferably 1,000 to 30,000 IU. When lipase is used, the content of lipase is 5,000 U to 300,000 U preferably 10,000 to 200,000 U.

The content of the bile acid compound is 200 mg to 10 g preferably 500 mg to 5 g.

When the above coloring system is employed, the content of cholesterol dehydrogenase is 200 to 30,000 U preferably 300 to 20,000 U. The content of the oxidized form coenzyme is 10 mg to 50 g preferably 30 mg to 20 g. The content of the color precursor is 5 mg to 10 g preferably 25 mg to 5 g, and the content of the electron transporting material is 1 mg to 1,000 mg preferably 5 mg to 500 mg. When diaphorase is employed as the electron transporting material, the content of diaphorase is 500 to 20,000 U preferably 700 to 10,000 U.

The content of the alkyphenoxypolyglycidol is 200 mg to 10 g preferably 500 mg to 5 g.

The dry-type analytical element of the invention comprises at least one liquid-permeable layer, and has a porous spreading layer. The liquid-permeable layer may be porous spreading layer, reagent layer containing dye-forming layer, light-blocking layer, water absorption layer, or the like, however, the porous spreading layer is essential for the analytical element of the invention.

The analytical element may be multilayer, and it may contain various known layers, such as undercoat layer, water absorption layer, adhesive layer, reagent layer, light-blocking layer and filtering layer, in addition to the spreading layer and a light-transmissive water-impermeable support. Examples of such a multilayer analytical element are disclosed in USP 3,992,158 and Japanese Patent KOKAI No. 55-164356.

The multilayer analytical element containing a support of the invention includes the following embodiments.

(1) A spreading layer and a support. A water absorption layer may be provided therebetween.

(2) A spreading layer, a reagent layer and a support superposed in this order. A water absorption layer may be provided between the reagent layer and the support. The cholesterol ester hydrolase may be incorporated in either of the spreading layer or the reagent layer, however the spreading layer is preferable.

(3) A spreading layer, a first reagent layer, a second reagent layer and a support. A water absorption layer may be provided between the second reagent layer and the support. The cholesterol ester hydrolase may be incorporated in any layer of the spreading layer, the first reagent layer or the second reagent layer, however the first reagent layer or the spreading layer is preferable.

(4) A spreading layer, a light-blocking layer, a reagent layer and a support superposed in this order. A water absorption layer may be provided between the reagent layer and the support. The cholesterol ester hydrolase may be incorporated in either of the spreading layer or the reagent layer, however the spreading layer is preferable.

(5) A spreading layer, a first reagent layer, a light-blocking layer, a second reagent layer and a support superposed in this order. A water absorption layer may be provided between the second reagent layer and the support. The cholesterol ester hydrolase may be incorporated in any layer of the spreading layer, the first reagent layer or the second reagent layer, however the spreading layer or the first reagent layer is preferable.

In the above embodiments, an adhesive layer may be provided on the surface of the layer located under the spreading layer for laminating it. The adhesive layer may be provided between other layers. A filtering layer may be provided between the spreading layer or the reagent layer and the water absorption layer, between the spreading layer and the reagent layer, between the first reagent layer and the second reagent layer, or between the light-blocking layer and the reagent layer or the spreading layer.

As the light-transmissive water-impermeable support, a known support employed in an usual multilayer analytical element may be employed. Such a support is a sheet or a laminate having a thickness in the range from 50 $\mu$m to 1 mm, preferably from 80 $\mu$m to 0.3 mm, and being clear in the range from near-ultraviolet to near infrared regions. Such a sheet or a laminate may be made of a polyester (for example, polyethylene terephthalate or polycarbonate of bisphenol A), a cellulose ester (for example, cellulose triacetate or cellulose acetate propionate) or polystyrene. A known undercoat layer may be provided on the surface of the support in order to secure the adhesion of the layer provided on the support such as a reagent layer or a water absorption layer to the support. Instead of the undercoat layer, surface of the support may be treated by physical activation such as glow discharge or corona discharge or by chemical activation in order to raise adhesive force.

The spreading layer preferably has liquid metering action. This liquid metering action means that the liquid sample spotted on the surface of this layer uniformly spreads in the transverse direction at the rate of almost equal amount per unit area without uneven distribution of any component. The material constituting matrix of the spreading layer may be filter papers, nonwoven fabrics, woven fabrics such as plain weaves including broad cloth and poplin, knitted fabrics such as tricot, double tricot and milanese, glass fiber filter papers, membrane filters formed out of blushed polymer, three-dimensional lattice structure material or the like. Among them, fibrous materials such as woven fabrics and knitted fabrics are preferable. The spreading layers using the above materials are disclosed in the specifications of Japanese Patent KOKAI Nos. 55-164356, 57-66359 and 60-222769. It is preferable that greases adhered on woven fabrics and knitted fabrics during their manufactures are substantially removed by degreasing such as washing.

The reagent layer contains a part of or whole reagents, and it is non-porous and water-absorptive or microporous and water-permeable.

A hydrophilic polymer binder which is employed for the substantially non-porous and water-absorptive reagent layer functions as a medium for dissolving or dispersing the reagents uniformly. It also functions to absorb the water in a sample and to carry the analyte together with the water. The hydrophilic polymer binder absorbs water to swell, and swelling ratio at water absorption of the polymer binder suitable for the reagent layer is generally 1.5 to 20 times, preferably 2.5 to 15 times at 30°C. Examples of the hydrophilic polymer binder usable for the reagent layer are gelatin including alkali-treated gelatin, acid-treated gelatin and deionized gelatin, gelatin derivatives such as phthalated gelatin, agarose, pullulan, pullulan derivatives, polyacrylamide, polyvinyl alcohol and polyvinyl pyrrolidone. Thickness of the reagent layer in dry state is usually 1 $\mu$m to 100 $\mu$m, preferably 3 $\mu$m to 30 $\mu$m.

The microporous and water-permeable reagent layer comprises a microporous structure layer constructed by solid particulates and a hydrophilic polymer binder as the binder thereof and reagent(s) or a reagent composition contained therein. The microporous structure layer referred herein has continuous microspaces structure composed of microporous or non-porous particulates and a hydrophilic polymer binder joining them.

Examples of the microporous or non-porous particulates include cellulose particulates such as microcrystalline cellulose and cellulose fine powder, particulates of silicon dioxide compound such as silica and diatomaceous earth, silicate particulates such as zeolite, polymer particulates, glass particulates, and various ceramic particulates. The hydrophilic polymer binder may be selected from the illustrated above or aqueous latex of copolymer containing more than 2% of hydrophilic repeating unit disclosed in EP 0,115,873 A. Thickness of the microporous reagent layer in dry state is 7 $\mu$m to 50 $\mu$m, preferably 10 $\mu$m to 30 $\mu$m.

The reagent layer may be incorporated with a pH buffer composition, a macromolecular pH buffer, a base polymer, an acid polymer, a macromolecular mordant, etc., to be known. The pH buffer composition may be a carbonate buffer, a borate buffer, a phosphate buffer, Good's buffer or the like.

The light-blocking layer is water-transmissive or water-permeable, and light-shielding particulates or the particulates having both functions of light-shielding and light-reflecting are disposed in and held by a small amount of a hydrophilic polymer binder capable of forming a film. The light-blocking layer shields color of an aqueous liquid sample, particularly red color of hemoglobin in a whole blood sample, spotted on the spreading layer during measuring the color development from the side of the light-transmissive support. This layer may also function as a light-reflecting layer or a background layer.

Examples of the particulates having both functions of light-shielding and light-reflecting are titanium dioxide particulates such as rutile, anatase and brookite microcrystalline particulates having a particle size of 0.1 $\mu$m to 1.2 $\mu$m, barium sulfate particulates and aluminum particulates and microflakes. Examples of the light-shielding particulates are carbon black, gas black and carbon microbeads. Among them, titanium dioxide particulates and barium sulfate particulates are preferable. The hydrophilic polymer binder having film-forming property may be selected from those illustrated in the foregoing reagent layer, weakly hydrophilic regenerated cellulose and cellulose acetate. Among them, gelatin, a gelatin derivative and polyacrylamide are preferable. To gelatin and a gelatin derivative, a known curing agent (a cross-linking agent) may be blended.

The light-blocking layer is provided by applying an aqueous suspension of light-shielding particulates containing a hydrophilic polymer binder by means of a known method, and then dried. Volume ratio of light-shielding particulates to hydrophilic polymer binder in dry state are light-shielding particulates 10 : hydrophilic polymer binder 2.5- 7.5, preferably 3.0- 6.5. In the case that the light-shielding particulates are titanium dioxide particulates, the ratio by weight of polymer binder is about 0.6- 1.8, preferably about 0.8- 1.5, per 10 of titanium dioxide. Thickness of the light-blocking layer in dry state is 3 $\mu$m to about 30 $\mu$m, preferably 5 $\mu$m to 20 $\mu$m.

The water absorption layer is composed of a hydrophilic polymer binder, and it may contain a cationic, ampholytic or nonionic surfactant and a pH buffer composition. The hydrophilic polymer binders of both layers may be selected from the illustrated previously. Thickness of these layers in dry state are usually 1 $\mu$m to 100 $\mu$m, preferably 3 $\mu$m to 30 $\mu$m.

The adhesive layer usually comprises the hydrophilic polymer binder to adhere the spreading layer at the wetting state or the swelling state of the polymer. The hydrophilic polymer binder usable for the adhesive layer may be selected from the illustrated in the foregoing water absorption layer, and gelatin, a gelatin derivative and polyacrylamide are preferable. Thickness of the adhesive layer in dry state is 0.5 $\mu$m to 20 $\mu$m, preferably 1 pm to 10 $\mu$m. A surfactant may be added to the adhesive layer. A nonionic surfactant, particularly having a chain structure of 8-15 hydroxyethylene or hydroxypropylene units is preferable.

The adhesive layer may be provided by applying a solution containing the hydrophilic polymer binder and a surfactant added by request by means of a known method.

In the multilayer analytical element of the invention, the enzyme having the hydrolytic activity against cholesterol ester, the bile acid compound, cholesterol dehydrogenase and alkylphenoxypolyglycidol are preferably incorporated in a upper layer, and spreading layer is the most preferable. However, cholesterol dehydrogenase may be incorporated the layer just under the spreading layer such as adhesive layer, light-blocking layer or first reagent layer. While, the bile acid compound is preferably incorporated in spreading layer together with the enzyme having the hydrolytic activity against cholesterol ester and nonionic surfactant such as alkylphenoxypolyethoxyethanol. Oxidized form coenzyme, color precursor and electron transporting material, are preferably incorporated in an under layer such as reagent layer. pH buffer may be incorporated in any layer.

The integral multilayer analytical element manufactured by the invention is preferably cut into square or circular pieces having a side or diameter of 15 mm to 30 mm, and put in a slide frame disclosed in Japanese Patent KOKAI No. 57-63452, USP 4,169,751, USP 4,387,990, PCT application WO 83/00391, etc. to use.

The measurement is carried out as follows. 5 $\mu\ell$ to about 30 $\mu\ell$, preferably about 8 $\mu\ell$ to about 15 $\mu\ell$ of an aqueous sample is spotted on the spreading layer, and incubated at a definite temperature in the range of about 20°C to about 45°C for a prescribed time, if necessary. Thereafter, detectable variation such as color change or coloring in the multilayer analytical element is measured from the side of the support through reflection photometry, and the subject component in the sample is determined by the principle of colorimetry.

## EXAMPLES

### Example 1

A colorless transparent polyethylene terephthalate (PET) film having a thickness of 180 $\mu$m on which gelatin undercoating was provided was employed as the support. On the support, the following solution was applied, and dried to form a dye-forming layer having a dry thickness of 15 $\mu$m.

| | |
|---|---|
| Alkali-treated gelatin | 12 g |
| Nonylphenylglycidyl ether | 0.1 g |
| NAD | 0.8 g |
| Diaphorase | 2800 IU |
| NBT* | 0.2 g |
| Water | 95 ml |

\* 3,3'-(3.3'-dimethoxy-4,4'-biphenylene)bis[2-(p-nitrophenyl)-5-phenyl-2H tetrazolium chloride]

On the dye-forming layer, the following suspension was applied, and dried to form a light-blocking layer having a thickness of 7 $\mu$m.

6

| Alkali-treated gelatin | 14 g |
|---|---|
| Rutile type titanium dioxide particulates | 70 g |
| Water | 100 ml |

On the light-blocking layer, an aqueous gelatin solution was applied, and dried to form an adhesive layer having a thickness of 2 $\mu$m.

The adhesive layer was dampened with 30 g/m$^2$ of water. A PET tricot fabric cloth having 0.25 mm in thickness was made hydrophilic by glow discharge, and pressed to laminate thereon as a spreading layer, and then dried.

On the spreading layer, the following suspension was applied at the rate of 200 ml/m$^2$, and dried to form an integral multilayer analytical element for the determination of cholesterol.

| Ethanol | 500 ml |
|---|---|
| Polyvinylpyrrolidone K90 | 18 g |
| Polyoxyethylene nonylphenyl ether (n = 40) | 6 g |
| 2-Amino-2-ethyl-1,3-propanediol | 11 g |
| Polyoxyethylene octylphenyl ether (n = 10) | 3 g |
| Sodium deoxycholate | 7 g |

The following enzyme solution was added to the above ethanol solution to obtain a suspension.

| Lipase[*] | 15000 U |
|---|---|
| Cholesterol dehydrogenase | 5000 U |
| Water | 5 ml |

* Produced by Chromobacterium viscosum

The analytical element thus obtained was cut into square pieces of 15 x 15 mm, and each piece was placed in a plastic mount.

Each 10 $\mu\ell$ of human sera having various cholesterol concentrations was spotted on the above analytical element, and incubated at 37°C for 6 minutes. Then, reflection optical density was measured by using light of 600 nm from the PET support side. The results are shown in Table 1.

Table 1

| Cholesterol Concentration | Reflection Optical Density |
|---|---|
| 62 mg/dl | 0.254 |
| 95 | 0.321 |
| 158 | 0.412 |
| 294 | 0.653 |
| 348 | 0.721 |

Example 2

The following solution was applied on a PET film having a thickness of 180 $\mu$m on which gelatin undercoating was provided, and dried to form a dye-forming layer having a dry thickness of 15 $\mu$m.

7

| Alkali-treated gelatin | 12 g |
|---|---|
| Nonylphenylglycidyl ether | 0.1 g |
| Diaphorase | 3000 U |
| NAD | 0.35 g |
| Bis(vinylsulfonylmethyl) ether | 0.12 g |
| Water | 105 g |

On the dye-forming layer, the following suspension was applied, and dried to form a light-blocking layer having a dry thickness of 7 $\mu$m.

| Alkali-treated gelatin | 7 g |
|---|---|
| Rutile type titanium dioxide particulates | 35 g |
| Water | 50 ml |

On the light-blocking layer, the following solution was applied, and dried to form an adhesive layer containing cholesterol dehydrogenase having a thickness of 4 $\mu$m.

| Cholesterol dehydrogenase | 39000 U |
|---|---|
| NAD | 0.9 g |
| Alkali-treated gelatin | 20 g |
| Nonylphenylglycidyl ether | 0.2 g |
| Water | 220 g |

The adhesive layer was dampened with 30 g/m$^2$ of water. A PET tricot fabric cloth having 0.25 mm in thickness was made hydrophilic by glow discharge, and pressed to laminate thereon as a spreading layer, and then dried.

On the spreading layer, the following suspension was applied at the rate of 200 ml/m$^2$, and dried to form an integral multilayer analytical element for the determination of cholesterol.

| Ethanol | 500 ml |
|---|---|
| Polyvinylpyrrolidone K90 | 18 g |
| Polyoxyethylene nonylphenyl ether (n = 40) | 6 g |
| 2-Amino-2-methyl-1,3-propanediol | 11 g |
| Polyoxyethylene octylphenyl ether (n = 10) | 3 g |
| Sodium deoxycholate | 7 g |

The following enzyme solution was added to the above ethanol solution to obtain a suspension.

| Lipase[*] | 15000 U |
|---|---|
| Water | 3 g |

* Produced by Chromobacterium viscosum

The analytical element thus obtained was cut into square pieces of 15 x 15 mm, and each piece was placed in a plastic mount.

Each 10 $\mu\ell$ of the human sera measured in Example 1 was spotted on the above analytical element, and the reflection optical density of each analytical element was measured in the same manner as Example 1. The results are shown in Table 2.

Table 2

| Cholesterol Concentration | Reflection Optical Density |
|---|---|
| 62 mg/dl | 0.330 |
| 95 | 0.402 |
| 158 | 0.565 |
| 294 | 0.928 |
| 348 | 1.08 |

Example 3

12,000 U of cholesterol esterase was used instead of 15,000 U of lipase, and an analytical element for the determination of cholesterol was prepared in the same manner as Example 2. In the case of this analytical element, similar results to Example 2 were obtained.

Example 4

In Example 1, 0.3 g of nonylphenoxypolyglycidol (n = 10) was used instead of 0.1 g of nonylphenylglycidyl ether in the solution for dye-forming layer, and 3 g of nonylphenoxypolyglycidol (n = 10) was used instead of 3 g of polyoxyethylene octylphenyl ether (n = 10) in the solution for applying on the spreading layer. Thus, an integral multilayer analytical element for the determination of cholesterol was prepared in the same manner as Example 1.

This analytical element was cut into square pieces of 15 x 15 mm, and each piece was placed in a plastic mount.

Each 10 $\mu\ell$ of the human sera measured in Example 1 was spotted on the above analytical element, and the reflection optical density of each analytical element was measured in the same manner as Example 1. The results are shown in Table 3.

Table 3

| Cholesterol Concentration | Reflection Optical Density |
|---|---|
| 62 mg/dl | 0.26 |
| 95 | 0.32 |
| 158 | 0.41 |
| 294 | 0.65 |
| 348 | 0.72 |

Example 5

In Example 2, 0.1 g of nonylphenylpolyglycidol and 0.35 g of NBT were employed instead of 0.1 g of nonylphenylglycidyl ether and 0.35 g of NAD in the solution for dye-forming layer, and 0.2 g of nonylphenylpolyglycidol (n = 10) was employed instead of 0.2 g of nonylphenylglycidyl ether in the solution for adhesive layer. Furthermore, 3 g of nonylphenylpolyglycidol (n = 10) was employed instead of 3 g of polyoxyethylene octylphenyl ether (n = 10) in the solution for applying on the spreading layer. Thus, an integral multilayer analytical element for the determination of cholesterol was prepared in the same manner as Example 2.

This analytical element was cut into square pieces of 15 x 15 mm, and each piece was placed in a plastic mount. Each 10 $\mu\ell$ of the human sera measured in Example 1 was spotted on the above analytical element, and the reflection optical density of each analytical element was measured in the same manner as Example 1. The results are shown in Table 4.

Table 4

| Cholesterol Concentration | Reflection Optical Density |
|---|---|
| 62 mg/dl | 0.330 |
| 95 | 0.402 |
| 158 | 0.565 |
| 294 | 0.928 |
| 348 | 1.08 |

Example 6

12,000 U of cholesterol esterase was used instead of 15,000 U of lipase, and an analytical element for determination of cholesterol was prepared in the same manner as Example 5. In the case of this analytical element, similar results to Example 5 were obtained.

**Claims**

1. A dry-type analytical element for the determination of cholesterol, comprising at least one liquid-permeable layer and having a porous spreading layer which is a liquid-permeable layer, and containing an enzyme having hydrolytic activity against cholesterol ester, a bile acid compound and cholesterol dehydrogenase in the same or different liquid-permeable layers.

2. The dry-type analytical element of claim 1 wherein said enzyme having hydrolytic activity against cholesterol ester is cholesterol esterase which is incorporated in said porous spreading layer.

3. The dry-type analytical element of claim 1 wherein said enzyme having hydrolytic activity against cholesterol ester is lipase which is incorporated in said porous spreading layer.

4. The dry-type analytical element of claim 3 wherein said lipase is produced by Chromobacterium viscosum.

5. The dry-type analytical element of claim 1 wherein said bile acid compound is incorporated in said porous spreading layer.

6. The dry-type analytical element of claim 5 wherein said bile acid compound is deoxycholic acid or its salt.

7. The dry-type analytical element of claim 1 wherein said cholesterol dehydrogenase is incorporated in said porous spreading layer.

8. The dry-type analytical element of claim 3 wherein said bile acid component is also incorporated in said porous spreading layer.

9. The dry-type analytical element of claim 8 wherein said cholesterol dehydrogenase is further incorporated in said porous spreading layer.

10. The dry-type analytical element of claim 8 or claim 9 wherein said lipase is produced by Chromobacterium viscosum.

11. The dry-type analytical element of claim 1 which further contains an alkylphenoxypolyglycidol in said liquid-permeable layer.

**Patentansprüche**

1. Analytisches Element des Trocken-Typs für die Bestimmung von Cholesterin, das mindestens eine flüssigkeitspermeable Schicht enthält und eine poröse Ausbreitungsschicht, die eine flüssigkeitspermeable Schicht ist, aufweist und ein Enzym mit hydrolytischer Aktivität gegenüber Cholesterinester, eine Gallensäureverbindung und Cholesterin-Dehydrogenase in der gleichen oder in unterschiedlichen flüssigkeitspermeablen Schicht(en) enthält.

2. Analytisches Element des Trocken-Typs nach Anspruch 1, wobei das Enzym, das hydrolytische Aktivität gegenüber Cholesterinester aufweist, Cholesterin-Esterase ist, die in die poröse Ausbreitungsschicht eingearbeitet ist.

3. Analytisches Element des Trocken-Typs nach Anspruch 1, wobei das Enzym mit hydrolytischer Aktivität gegenüber Cholesterinester Lipase ist, die in die poröse Ausbreitungsschicht eingearbeitet ist.

4. Analytisches Element des Trocken-Typs nach Anspruch 3, wobei die Lipase durch Chromobacterium viscosum gebildet wird.

5. Analytisches Element des Trocken-Typs nach Anspruch 1, wobei die Gallensäureverbindung in die poröse Ausbreitungsschicht eingearbeitet ist.

6. Analytisches Element des Trocken-Typs nach Anspruch 5, wobei die Gallensäureverbindung Desoxycholsäure oder ihr Salz ist.

7. Analytisches Element des Trocken-Typs nach Anspruch 1, wobei die Cholesterin-Dehydrogenase in die poröse Ausbreitungsschicht eingearbeitet ist.

8. Analytisches Element des Trocken-Typs nach Anspruch 3, wobei die Gallensäurekomponente ebenfalls in die poröse Ausbreitungsschicht eingearbeitet ist.

9. Analytisches Element des Trocken-Typs nach Anspruch 8, wobei die Cholesterin-Dehydrogenase zusätzlich in die poröse Ausbreitungsschicht eingearbeitet ist.

10. Analytisches Element des Trocken-Typs nach Anspruch 8 oder 9, wobei die Lipase durch Chromobacterium viscosum gebildet wird.

11. Analytisches Element des Trocken-Typs nach Anspruch 1, welches zusätzlich ein Alkylphenoxypolyglycidol in der flüssigkeitspermeablen Schicht enthält.

**Revendications**

1. Elément analytique de type sec destiné à la détermination du cholestérol, comprenant au moins une couche perméable aux liquides et ayant une couche d'étalement poreuse qui est une couche perméable aux liquides, et contenant une enzyme ayant une activité hydrolytique vis-à-vis des esters de cholestérol, un composé d'acide biliaire et de la cholestérol-déshydrogénase dans les mêmes couches ou des couches différentes perméables aux liquides.

2. Elément analytique de type sec selon la revendication 1, dans lequel ladite enzyme ayant une activité hydrolytique vis-à-vis des esters de cholestérol est la cholestérol-estérase qui est incorporée dans ladite couche d'étalement poreuse.

3. Elément analytique de type sec selon la revendication 1, dans lequel ladite enzyme ayant une activité hydrolytique vis-à-vis des esters de cholestérol est la lipase qui est incorporée dans ladite couche d'étalement poreuse.

4. Elément analytique de type sec selon la revendication 3, dans lequel ladite lipase est produite par Chromobacterium viscosum.

5. Elément analytique de type sec selon la revendication 1, dans lequel ledit composé de l'acide biliaire est incorporé dans ladite couche d'étalement poreuse.

6. Elément analytique de type sec selon la revendication 5, dans lequel ledit composé de l'acide biliaire est l'acide désoxycholique ou son sel.

7. Elément analytique de type sec selon la revendication 1, dans lequel ladite cholestérol-déshydrogénase est incorporée dans ladite couche d'étalement poreuse.

8. Elément analytique de type sec selon la revendication 3, dans lequel ledit composant de l'acide biliaire est également incorporé dans ladite couche d'étalement poreuse.

9. Elément analytique de type sec selon la revendication 8, dans lequel ladite cholestérol-déshydrogénase est en outre incorporée dans ladite couche d'étalement poreuse.

10. Elément analytique de type sec selon la revendication 8 ou la revendication 9, dans lequel ladite lipase est produite par Chromobacterium viscosum.

11. Elément analytique de type sec selon la revendication contenant en outre un alkylphénoxypolyglycidol dans ladite couche perméable aux liquides.